# EUROPEAN PATENT APPLICATION

(11) **EP 3 241 562 A1**
(43) Date of publication of application: **08.11.2017**
(21) Application number: 16382191.1
(22) Date of filing: 02.05.2016
(51) Int. Cl.: A61K 45/06, A61K 31/423, A61P 35/00

(54) **ZONISAMIDE FOR USE IN THE TREATMENT OF BREAST CANCER**

(71) Applicant: Fundació Institut Mar d'Investigacio Medica, 08003 Barcelona (ES)
(72) Inventor: PEIRÓ SALES, Sandra, 08008 Barcelona (ES); MESTRES LÓPEZ, Jordi, 08003 Barcelona (ES); CEBRIÀ COSTA, Joan Pau, 08003 Barcelona (ES); ITURBIDE MARTÍNEZ DE ALBÉNIZ, Ane, 08003 Barcelona (ES); ANTOLIN HERNANDEZ, Albert, 08003 Barcelona (ES)
(74) Representative: Gallego Jiménez, José Fernando

(57) **Abstract**

The present invention relates to a new use of zonisamide, namely to zonisamide for use in the treatment breast cancer, in particular triple negative breast cancer tumours. It relates also to a pharmaceutical composition comprising zonisamide and at least one pharmaceutically acceptable excipient or vehicle, for use in the treatment of breast cancer.

## Description

### Technical Field

The present invention relates to zonisamide for use in the treatment of breast cancer.

### Technical Background

Breast cancer is the most common cancer in women worldwide with nearly 1.7 million new cases diagnosed in 2012. This represents about 12% of all new cancer cases and 25% of all cancers in women. In 2012, the estimated incidence rate in Europe was 71.1 per 100,000 women and a mortality rate of 16.1 per 100,000 women. While only approximately 5% of all newly diagnosed breast cancer patients in the U.S. present with metastatic disease at the time of initial diagnosis, up to one third of patients with early stage disease will subsequently develop metastasis.

Over the past two decades, breast cancer mortality rates have declined by about 30%, with corresponding improvements in 5-year overall survival rates to 90%. Despite these advances, metastatic breast cancer (MBC) remains incurable, with an estimated 5-year overall survival rate below 25%.

Breast cancer traditionally has been classified into three different subtypes based on the presence or absence of three receptors found on cancer cells. Hormone receptor (HR) positive breast cancers express estrogen and/or progesterone receptors (ER/PR), and constitute approximately 60% of all breast cancer cases. The oncogene human epidermal growth factor receptor 2 (HER-2/neu) is over-expressed in approximately 20% of all breast cancer cases; while approximately 20% of breast cancer cases are negative for the expression of ER, PR, and HER-2/neu, also known as triple negative breast cancer.

Patients with hormone receptor positive tumors typically receive endocrine therapy (e.g. selective estrogen-receptor response modulators [SERM] and aromatase inhibitors [AI]) as one of many options of their treatment. Moreover, patients with HER-2/neu overexpressing tumors typically receive anti-HER/2 targeted therapy in combination with cytotoxic chemotherapeutic agents. Patients with triple negative breast cancer do not have these targeted treatment options, and it is typically treated with a combination of therapies such as surgery, radiation therapy, and chemotherapy, being cytotoxic chemotherapy the primary modality.

Triple negative breast cancers are typically associated with poor prognosis, due to aggressive tumor phenotype(s), only partial response to chemotherapy and present lack of clinically established targeted therapies and represent the focus of increasing interest at the clinical, biological and epidemiological level, as disclosed in Podo et al., Triple-negative breast cancer: Present challenges and new perspectives, Mol. Oncol., 2010, 4, 209-229.

There is, thus, a particular need for new active agents suitable for breast cancer treatment, and in particular for triple negative breast cancers.

### Object of the invention

The object of the present invention is zonisamide for use in the treatment of breast cancer.

Also part of the invention is a pharmaceutical composition comprising zonisamide for use in the treatment of breast cancer.

### Brief description of drawings:

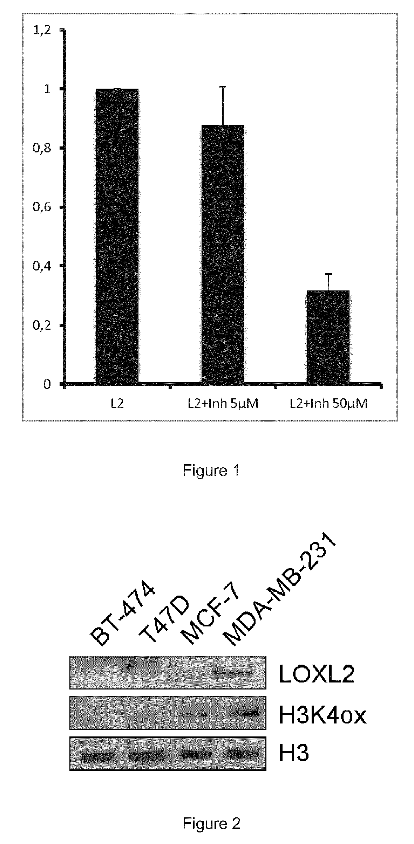

Figure 1 discloses a representation of the reduction of the hydrogen peroxide release in the presence of lysyl oxidase like-2 protein (LOXL2) with the histone H3 peptide trimethylated in lysine 4 (H3K4me3) and increasing concentrations of zonisamide was measured by fluorescence using the Amplex^{®} Red assay, as disclosed in Example 1. In the ordinate axis it is represented the fold change of hydrogen peroxide production in arbitrary units. In the abscissa axis are represented the assays corresponding to incubations in the absence of zonisamide, and in the presence of 5 µM and 50 µM final concentrations of zonisamide. It is observed a reduction in the production of hydrogen peroxide by increasing the concentration of zonisamide.
Figure 2 refers to a picture of the analysis of cell lysates from the different breast cancer cell lines (BT-474, T-47D, MCF-7 and MDA-MB-231) by western blot showing the levels of LOXL2 and oxidized H3 (H3K4ox). It is observed that breast cancer triple negative human cells (MDA-MB-231) show high levels of LOXL2, which correlate with an enrichment of H3K4ox, the product produced by LOXL2 by oxidation of H3K4me3.

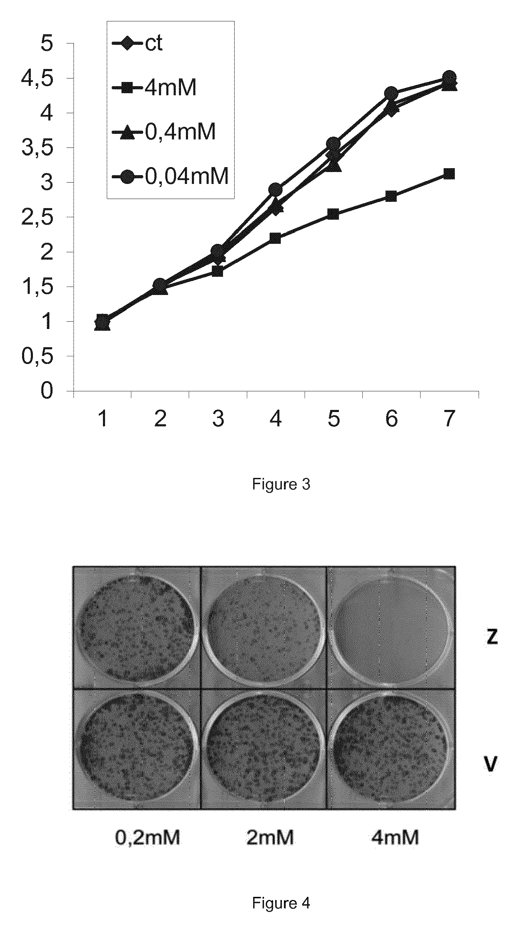

Figure 3 discloses the effect of zonisamide on growth of breast cancer triple negative human cells (MDA-MB-231). In the ordinate axis it is represented the absorbance at 570 nm. In the abscissa axis are represented the time line expressed in days. The series represent the control without zonisamide (◆), and increasing concentrations of zonisamide 0.04 mM (▲), 0.4 mM (●) and 4 mM (■). It is observed an inhibition of LOXL2 activity by increasing the concentration of zonisamide.
Figure 4 shows a picture of plates with colonies containing breast cancer triple negative human cells (MDA-MB-231), treated at different final concentrations of zonisamide (0.2 mM, 2 mM and 4 mM), and plates with colonies treated only with vehicle DMSO. It was observed that inhibition of LOXL2 activity decreases the proliferative capacity of these cells and causes cell death

### Detailed description of the invention

The object of the present invention is zonisamide, a pharmaceutically acceptable salt thereof, a solvate thereof or a prodrug thereof, for use in an effective amount in the treatment of breast cancer, preferably in the treatment of triple negative breast cancer.

Alternatively the object of the invention can also be formulated as the use of an effective amount of zonisamide, a pharmaceutically acceptable salt thereof, a solvate thereof or a prodrug thereof, for the preparation of a medicament for the treatment of breast cancer, preferably the treatment of triple negative breast cancer.

Alternatively the object of the invention can also be formulated as a method for the treatment of breast cancer, preferably the treatment of triple negative breast cancer, comprising administering an effective amount of zonisamide, a pharmaceutically acceptable salt thereof, a solvate thereof or a prodrug thereof, to a patient in need of treatment for this disease.

The authors of the present invention have discovered that zonisamide inhibits the enzymatic activity of lysyl oxidase like-2 protein (LOXL2), and consequently opens a therapeutic window for the treatment of breast cancer, in particular triple negative breast cancer.

Triple-negative breast cancers (TNBC) are characterized by absence of estrogen receptor (ER), progesterone receptor (PR) and lack of overexpression of human epidermal growth factor receptor 2 (HER2).

In the present description as well as in the claims, the singular forms "a" and "an" include also the plural reference unless the context clearly indicates otherwise.

### Zonisamide

Zonisamide is the INN of compound benzo[d]isoxazol-3-ylmethanesulfonamide, which responds to the structure:

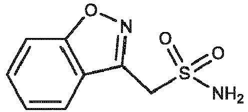

Zonisamide is a benzisoxazole derivative, which can be prepared according to the process disclosed in, for example, US patent 4,172,896.

It is used as medicament as a broad-spectrum antiepileptic drug (AED) that has been shown to be effective in the treatment of refractory partial seizures.

Surprisingly it has been found that zonisamide inhibits *in vitro* the enzymatic activity of human recombinant LOXL2 enzyme, and that such inhibition decreases the proliferative capacity of breast cancer cells *in vivo* and causes cell death. Breast cancer triple negative cells with high LOXL2 levels died after 4 days of zonisamide treatment.

In the present invention the term "zonisamide" refers to zonisamide, a pharmaceutically acceptable salt thereof, a solvate thereof, or a prodrug thereof.

Among the pharmaceutically acceptable salts of zonisamide it can be mentioned, for example, quaternized amine salts, alkali metal salts (e.g. sodium and potassium salt) and alkaline earth metal salts (e.g. magnesium and calcium salt).

In addition, zonisamide or a salt thereof may be used in the same manner in its solvate form. According to the present invention, "solvate" is preferably a pharmacologically acceptable solvate. The pharmacologically acceptable solvate may be either a hydrate or a nonhydrate, preferably a hydrate. As a nonhydrate, alcohols (e.g., ethanol, *n*-propanol), dimethylformamide (DMF), dimethylsulfoxide (DMSO) or else can be used.

Moreover, zonisamide, a salt thereof or a solvate thereof may be used in the same manner in its prodrug form. The term "prodrug" refers to "an active form of a drug" (i.e., the "drug" corresponding to the prodrug) that is chemically modified into an inactive substance for the purpose of improving bioavailability, alleviating side-effects or else, which is absorbed in the body and then metabolized into the active form to exert action. Thus, the term "prodrug" refers to any compound having a lower intrinsic activity than a corresponding "drug", which, once administered into a biological system, generates the "drug" that provides specific activity through spontaneous chemical reaction or enzymatic catalysis or metabolic reaction. Examples of such prodrugs include compounds in which a sulfamoyl group or the like of zonisamide is acylated, alkylated, phosphorylated, borated, carbonated, esterified, amidated or urethanated. This exemplified group, however, is not comprehensive, but merely a typical group of examples and those skilled in the art can prepare other known prodrugs from zonisamide according to a known method. Prodrugs produced from zonisamide are within the scope of the present invention.

Zonisamide can be used alone, or in combination with other active agents, for the treatment of breast cancer, preferably the triple negative breast cancer. In a preferred embodiment, zonisamide is for use alone. In another preferred embodiment zonisamide is for use in combination with other agents. The other agents include but are not limited to anastrozole, bevacizumab, capecitabine, carboplatin, cisplatin, cyclophosphamide, daunorubicin, denosumab, docetaxel, doxorubicin, epirubicin, epoetin alfa, eribulin, everolimus, exemestane, filgrastim, fluorouracil, fluoxymesterone, folinic acid, fulvestrant, gemcitabine, goserelin, iniparib, ixabepilone, lapatinib, letrozole, leuprolide, megestrol, methotrexate, mitoxantrone, mutamycin, olaparib, paclitaxel, palbociclib, pamidronate, pegfilgrastim, pertuzumab, raloxifene, sorafenib, sunitinib, tamoxifen, thiotepa, toremifene, transtuzumab, trastuzumab emtansine, triptorelin, vinblastine, vincristine, vinorelbine, zoledronic acid, pharmaceutically acceptable salts thereof, and mixtures thereof. If for use in combination with other active agents, zonisamide is preferably combined with bevacizumab, carboplatin, cisplatin, cyclophosphamide, docetaxel, doxorubicin, iniparib, olaparib, paclitaxel, pharmaceutically acceptable salts thereof, and mixtures thereof.

Examples of pharmaceutically acceptable salts include pharmaceutically acceptable acid addition salts, pharmaceutically acceptable base addition salts, pharmaceutically acceptable metal salts, ammonium and alkylated ammonium salts. Acid addition salts include salts of inorganic acids as well as organic acids. Representative examples of suitable inorganic acids include hydrochloric, hydrobromic, hydroiodic, phosphoric, sulfuric, nitric acids and the like. Representative examples of suitable organic acids include formic, acetic, trichloroacetic, trifluoroacetic, propionic, benzoic, cinnamic, citric, fumaric, glycolic, lactic, maleic, malic, malonic, mandelic, oxalic, picric, pyruvic, salicylic, succinic, methanesulfonic, ethanesulfonic, tartaric, ascorbic, pamoic, bismethylene salicylic, ethanedisulfonic, gluconic, citraconic, aspartic, stearic, palmitic, EDTA, glycolic, *p*-aminobenzoic, glutamic, benzenesulfonic, *p*-toluenesulfonic acids, sulphates, nitrates, phosphates, perchlorates, borates, acetates, benzoates, hydroxynaphthoates, glycerophosphates, ketoglutarates and the like. Examples of metal salts include lithium, sodium, potassium, magnesium salts and the like. Examples of ammonium and alkylated ammonium salts include ammonium, methylammonium, dimethylammonium, trimethylammonium, ethylammonium, hydroxyethylammonium, diethylammonium, butylammonium, tetramethylammonium salts and the like. Examples of organic bases include lysine, arginine, guanidine, diethanolamine, choline and the like.

### Composition

Also part of the invention is a pharmaceutical composition comprising an effective amount of zonisamide, a pharmaceutically acceptable salt thereof, a solvate thereof or a prodrug thereof, and at least one pharmaceutically acceptable excipient or vehicle, for use in the treatment of breast cancer, in particular triple negative breast cancer.

Alternatively this aspect of the invention can also be formulated as the use of a pharmaceutical composition comprising an effective amount of zonisamide, a pharmaceutically acceptable salt thereof, a solvate thereof or a prodrug thereof, and at least one pharmaceutically acceptable excipient or vehicle for the preparation of a medicament for the treatment of breast cancer, in particular triple negative breast cancer.

Alternatively this aspect of the invention can also be formulated as a method for the treatment of breast cancer, preferably the treatment of triple negative breast cancer, comprising administering a pharmaceutical composition comprising an effective amount of zonisamide, a pharmaceutically acceptable salt thereof, a solvate thereof or a prodrug thereof, and at least one pharmaceutically acceptable excipient or vehicle, to a patient in need of treatment for this disease.

Further, a pharmaceutical composition of zonisamide with one or more of the other active agents listed above are also included in the invention, for use in the treatment of breast cancer, in particular triple negative breast cancer.

Also included in the present invention is a pharmaceutical composition containing zonisamide and an additional active agent selected from the above list, adapted for simultaneous, separate or sequential use in the treatment of breast cancer, in particular triple negative breast cancer.

Pharmaceutical compositions suitable for the delivery zonisamide or combinations thereof with the above mentioned active ingredients and methods for their preparation will be readily apparent to those skilled in the art. Such compositions and methods for their preparation may be found, for example, in Remington The Science and Practice of Pharmacy, 20th Edition ed. A. R. Gennaro, Lippincott Williams & Wilikins, 2000.

Excipients and vehicles suitable for the preparation of those pharmaceutical compositions are known to the skilled person in the art, and can be found in Handbook of Pharmaceutical Excipients, 4th Edition, Ed. R. C. Crove, P. J. Sheskey and P. J. Weller, Pharmaceutical Press, 2003.

Any suitable route of administration may be employed for providing a mammal, especially a human, with an effective dosage of zonisamide. For example, oral (including buccal and sublingual administration), rectal, topical including ocular and nasal administration), parenteral, pulmonary, and the like may be employed. Dosage forms include tablets, troches, dispersions, suspensions, solutions, capsules, creams, ointments, aerosols, and the like. Preferably, zonisamide is administered orally or parentally.

The effective dosage of active ingredient employed may vary depending on the mode of administration, the condition being treated and the severity of the condition being treated. Such dosage may be ascertained readily by a person skilled in the art. Generally, zonisamide is administered from 1 to 600 mg per day, preferably 25 to 400 mg per day, and more preferably 50 to 300 mg per day. The total daily dose may be administered in single or divided doses and may, at the physician's discretion, fall outside of the typical range given herein.

These dosages are based on an average human subject having a weight of about 65 kg to 70 kg. The physician will readily be able to determine doses for subjects whose weight falls outside this range.

As mentioned, zonisamide may be administered orally. Oral administration may involve swallowing, so that the active ingredient enters the gastrointestinal tract, and/or buccal, lingual or sublingual administration by which the active ingredient enters the blood stream directly from the mouth.

Formulations suitable for oral administration include solid, semi-solid and liquid systems such as tablets; soft or hard capsules containing multi- or nano-particulates, liquids, or powders; lozenges (including liquid-filled); chews; gels; fast dispersing dosage forms; films; ovules; sprays; and buccal/mucoadhesive patches.

Liquid formulations include suspensions, solutions, syrups and elixirs. Such formulations may be employed as fillers in soft or hard capsules (made, for example, from gelatin or hydroxypropylmethylcellulose) and typically comprise a carrier, for example, water, ethanol, propylene glycol, polyethylene glycol, methylcellulose, or a suitable oil, and one or more emulsifying agents and/or suspending agents. Liquid formulations may also be prepared by the reconstitution of a solid, for example, from a sachet.

Zonisamide may also be used in fast-dissolving, fast-disintegrating dosage forms.

For tablet dosage forms, depending on dose, the active agent may make up from 1 wt% to 80 wt% of the dosage form, more typically from 5 wt% to 60 wt% of the dosage form. In addition to the active agent, tablets generally contain a disintegrant. Examples of disintegrants include sodium carboxymethyl cellulose, sodium starch glycolate, calcium carboxymethylcellulose, croscarmellose sodium, crospovidone, polyvinylpyrrolidone, methylcellulose, microcrystalline cellulose, lower alkyl-substituted hydroxypropylcellulose, starch, pregelatinised starch and sodium alginate. Generally, the disintegrant will comprise from 1 wt% to 25 wt%, preferably from 5 wt% to 20 wt% of the dosage form.

Binders are generally used to impart cohesive qualities to a tablet formulation. Suitable binders include gelatin, microcrystalline cellulose, sugars, polyethylene glycol, natural and synthetic gums, polyvinylpyrrolidone, pregelatinised starch, hydroxypropylcellulose and hydroxypropylmethylcellulose. Tablets may also contain diluents, such as lactose (monohydrate, spray-dried monohydrate, anhydrous and the like), sucrose, sorbitol, mannitol, xylitol, dextrose, microcrystalline cellulose, starch and dibasic calcium phosphate dihydrate.

Tablets may also optionally comprise surface active agents, such as polysorbate 80, sodium lauryl sulfate, and glidants such as silicon dioxide and talc. When present, surface active agents may comprise from 0.2 wt% to 5 wt% of the tablet, and glidants may comprise from 0.2 wt% to 1 wt% of the tablet.

Tablets also generally contain lubricants such as magnesium stearate, calcium stearate, zinc stearate, sodium stearyl fumarate, and mixtures of magnesium stearate with sodium lauryl sulphate. Lubricants generally comprise from 0.25 wt% to 10 wt%, preferably from 0.5 wt% to 3 wt% of the tablet.

Other possible ingredients include anti-oxidants, colorants, flavourings and flavour enhancers, preservatives, salivary stimulating agents, cooling agents, cosolvents (including oils), emollients, bulking agents, anti-foaming agents, surfactants and taste-masking agents.

Exemplary tablets contain up to about 80% active agent, from about 10 wt% to about 90 wt% binder, from about 0 wt% to about 85 wt% diluent, from about 2 wt% to about 10 wt% disintegrant, and from about 0.25 wt% to about 10 wt% lubricant. Tablet blends may be compressed directly or by roller to form tablets. Tablet blends or portions of blends may alternatively be wet-, dry-, or melt-granulated, melt congealed, or extruded before tabletting. The final formulation may comprise one or more layers and may be coated or uncoated; it may even be encapsulated.

The formulation of tablets is discussed, for example, in Pharmaceutical Dosage Forms: Tablets. Vol. 1, by H. Lieberman, L. Lachman, J. B. Schwartz (2nd Edition, Marcel Dekker, New York, 1989).

Solid formulations for oral administration may be formulated to be immediate and/or modified release. Modified release formulations include delayed-, sustained-, pulsed-, controlled-, targeted and programmed release.

Controlled release compositions for oral use may, e.g., be constructed to release the active agent by controlling the dissolution and/or the diffusion thereof.

Dissolution or diffusion controlled release can be achieved by appropriate coating of a tablet, capsule, pellet, or granulate formulation of active agents, or by incorporating the active agent into an appropriate matrix. A controlled release coating may include one or more of the coating substances mentioned above and/or, e.g., shellac, beeswax, glycowax, castor wax, carnauba wax, stearyl alcohol, glyceryl monostearate, glyceryl distearate, glycerol palmitostearate, ethylcellulose, acrylic resins, dl-polylactic acid, cellulose acetate butyrate, polyvinyl chloride, polyvinyl acetate, vinyl pyrrolidone, polyethylene, polymethacrylate, methylmethacrylate, 2-hydroxymethacrylate, methacrylate hydrogels, 1,3-butylene glycol, ethylene glycol methacrylate, and/or polyethylene glycols. In a controlled release matrix formulation, the matrix material may also include, e.g., hydrated metylcellulose, carnauba wax and stearyl alcohol, carbopol 934, silicone, glyceryl tristearate, methyl acrylate-methyl methacrylate, polyvinyl chloride, polyethylene, and/or halogenated fluorocarbon.

A controlled release composition containing one or more of the active agents of the claimed combinations may also be in the form of a buoyant tablet or capsule (i.e., a tablet or capsule that, upon oral administration, floats on top of the gastric content for a certain period of time). A buoyant tablet formulation of the active agent(s) can be prepared by granulating a mixture of the active agent(s) with excipients and 20-75% w/w of hydrocolloids, such as hydroxyethylcellulose, hydroxypropylcellulose, or hydroxypropylmethyl-cellulose. The obtained granules can then be compressed into tablets. On contact with the gastric juice, the tablet forms a substantially water-impermeable gel barrier around its surface. This gel barrier takes part in maintaining a density of less than one, thereby allowing the tablet to remain buoyant in the gastric juice.

Zonisamide may also be administered parentally directly into the blood stream, into muscle, or into an internal organ. Suitable means for parenteral administration include intravenous, intraarterial, intraperitoneal, intrathecal, intraventricular, intraurethral, intrasternal, intracranial, intramuscular, intrasynovial and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile non-aqueous solution or as a dried form to be used in conjunction with a suitable vehicle such as sterile, pyrogen-free water.

The preparation of parenteral formulations under sterile conditions, for example, by lyophilisation, may readily be accomplished using standard pharmaceutical techniques well known to those skilled in the art. The solubility of zonisamide in parenteral solutions may be increased by the use of appropriate formulation techniques, such as the incorporation of solubility-enhancing agents.

Formulations for parenteral administration may be formulated to be immediate and/or modified release. Modified release formulations include delayed-, sustained-, pulsed-, controlled-, targeted and programmed release. Thus zonisamide and combinations with the above mentioned active ingredients may be formulated as a suspension or as a solid, semi-solid, or thixotropic liquid for administration as an implanted depot providing modified release of the active ingredient. Examples of such formulations include active agent-coated stents and semi-solids and suspensions comprising active agent-loaded poly(d/-lactic-coglycolic)acid (PGLA) microspheres.

Zonisamide may also be administered topically, (intra)dermally, or transdermally to the skin or mucosa. Typical formulations for this purpose include gels, hydrogels, lotions, solutions, creams, ointments, dusting powders, dressings, foams, films, skin patches, wafers, implants, sponges, fibres, bandages and microemulsions. Liposomes may also be used. Typical carriers include alcohol, water, mineral oil, liquid petrolatum, white petrolatum, glycerin, polyethylene glycol and propylene glycol.

Formulations for topical administration may be formulated to be immediate and/or modified release. Modified release formulations include delayed-, sustained-, pulsed-, controlled-, targeted and programmed release.

Zonisamide can also be administered intranasally or by inhalation, typically in the form of a dry powder (either alone, as a mixture, for example, in a dry blend with lactose, or as a mixed component particle, for example, mixed with phospholipids, such as phosphatidylcholine) from a dry powder inhaler or as an aerosol spray from a pressurised container, pump, spray, atomiser (preferably an atomiser using electrohydrodynamics to produce a fine mist), or nebuliser, with or without the use of a suitable propellant, such as 1 ,1 ,1 ,2- tetrafluoroethane or 1 ,1 ,1 ,2,3,3,3-heptafluoropropane, or as nasal drops. For intranasal use, the powder may comprise a bioadhesive agent, for example, chitosan or cyclodextrin.

The pressurised container, pump, spray, atomizer, or nebuliser contains a solution or suspension of zonisamide or a combination thereof with the above mentioned active ingredients comprising, for example, ethanol, aqueous ethanol, or a suitable alternative agent for dispersing, solubilising, or extending release of the active, a propellant(s) as solvent and an optional surfactant, such as sorbitan trioleate, oleic acid, or an oligolactic acid.

Prior to use in a dry powder or suspension formulation, the active agent is micronized to a size suitable for delivery by inhalation (typically less than 5 microns). This may be achieved by any appropriate comminuting method, such as spiral jet milling, fluid bed jet milling, supercritical fluid processing to form nanoparticles, high pressure homogenisation, or spray drying.

Capsules (made, for example, from gelatin or hydroxypropylmethylcellulose, blisters and cartridges for use in an inhaler or insufflator may be formulated to contain a powder mix of zonisamide , a suitable powder base such as lactose or starch and a performance modifier such as leucine, mannitol, or magnesium stearate. The lactose may be anhydrous or in the form of the monohydrate, preferably the latter. Other suitable excipients include dextran, glucose, maltose, sorbitol, xylitol, fructose, sucrose and trehalose.

Suitable flavours, such as menthol and levomenthol, or sweeteners, such as saccharin or saccharin sodium, may be added to those formulations intended for inhaled/intranasal administration.

Formulations for inhaled/intranasal administration may be formulated to be immediate and/or modified release using, for example, PGLA. Modified release formulations include delayed-, sustained-, pulsed-, controlled-, targeted and programmed release.

Zonisamide or a combination thereof with the above mentioned active ingredients may be administered rectally or vaginally, for example, in the form of a suppository, pessary, or enema. Cocoa butter is a traditional suppository base, but various alternatives may be used as appropriate.

Formulations for rectal/vaginal administration may be formulated to be immediate and/or modified release. Modified release formulations include delayed-, sustained-, pulsed-, controlled-, targeted and programmed release.

Zonisamide or a combination thereof with the above mentioned active ingredients may also be administered directly to the eye or ear, typically in the form of drops of a micronised suspension or solution in isotonic, pH-adjusted, sterile saline. Other formulations suitable for ocular and aural administration include ointments, gels, biodegradable (e.g. absorbable gel sponges, collagen) and non-biodegradable (e.g. silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose, or a heteropolysaccharide polymer, for example, gellan gum, may be incorporated together with a preservative, such as benzalkonium chloride. Such formulations may also be delivered by iontophoresis.

Formulations for ocular/aural administration may be formulated to be immediate and/or modified release. Modified release formulations include delayed-, sustained-, pulsed-, controlled-, targeted, or programmed release. Zonisamide may be combined with soluble macromolecular entities, such as cyclodextrin and suitable derivatives thereof or polyethylene glycol-containing polymers, in order to improve their solubility, dissolution rate, taste-masking, bioavailability and/or stability for use in any of the aforementioned modes of administration.

For the avoidance of doubt, references herein to "treatment" include references to curative, palliative and prophylactic treatment.

### In vitro tests

The inhibition of the enzymatic activity of LOXL2 by zonisamide can be checked using *in vitro* tests, wherein human recombinant LOXL2 enzyme, purified using the baculovirus system as disclosed in Herranz et al., Lysyl oxidase-like 2 deaminates lysine 4 in histone H3, Molecular cell, 2012, 46, 369-376, is subsequently incubated with histone H3 peptide (SEQ ID NO: 1) trimethylated in lysine 4 (H3K4me3). This peptide is previously identified as a LOXL2 substrate (Herranz et al., Lysyl oxidase-like 2 deaminates lysine 4 in histone H3, Molecular cell, 2012, 46, 369-376).

One of the by-products generated during the oxidation reaction is H₂0₂ and the detection of this compound is used as readout of the catalytic activity of LOXL2. Hydrogen peroxide detection was evaluated using Amplex^{®} Red Hydrogen Peroxide/Peroxidase Assay Kit.

The presence of different concentrations of zonisamide in the oxidation reaction inhibites LOXL2 activity, represented by the reduction in the production of hydrogen peroxide, as seen in Figure 1.

### In vivo tests

The levels of LOXL2 and H3K4ox can be checked in an *in vivo* test, by western-blot in a panel of breast cancer cell lines. It is observed that breast cancer triple negative cells show high levels of LOXL2, which in turn correlate with an enrichment of H3K4ox, the product produced by LOXL2 as shown in Figure 2. As a result of LOXL2 oxidation, the trimethylated amino group of H3K4me3 peptide is released and K4 is converted to an allysine (H3K4ox).

In an *in vivo* test, MDA-MB-231, breast cancer triple negative cells can be treated with increasing concentrations of zonisamide for several days using both a MTT assay and a clonogenic assay. The MTT assay is a colorimetric assay for assessing cell metabolic activity. Specific enzymes are capable of reducing the tetrazolium dye MTT 3-(4,5-dimethylthiazol-2-yl)-2,5-diphenyltetrazolium bromide to its insoluble formazan, which has a purple color. A clonogenic assay is a cell biology technique for studying the effectiveness of specific agents on the survival and proliferation of cells.

In the MTT assay it is be observed that an increase in the concentration of zonisamide produces a reduction in the LOXL2 activity, as shown in Figure 3.

In the clonogenic assay, MDA-MB-231 cells are treated with zonisamide at different concentrations. It is observed that inhibition of LOXL2 activity decreases the proliferative capacity of these cells and causes cell death as shown in Figure 4.

### Examples

### Materials

Zonisamide can be prepared according to the process disclosed in US patent 4,172,896.

Recombinant LOXL2 enzyme can be obtained as disclosed in Herranz *et al., op.cit.*

H3K4me3 peptide corresponds to HE peptide (SEQ ID NO: 1), which contains a trimethylated lysine in position 4.

MDA-MB-231 cells, Luminal A T-47D cells, Luminal A MCF-7 cells and Luminal B BT-474 cells can be obtained from the company ATCC (USA).

Antibodies anti-LOXL2 can be obtained from the company Novus Biologicals (USA). Antibodies anti-H3 can be obtained from the company Millipore (USA), and antibodies anti-H3K4ox from the Institut Hospital del Mar d'Investigacions Mèdiques (IMIM).

### Example 1: In vitro inhibition of the enzymatic activity of LOXL2 by zonisamide

In this *in vitro* test, human recombinant LOXL2 enzyme was purified using the baculovirus system as disclosed in Herranz *et al., op.cit.,* and subsequently incubated with histone H3 peptide trimethylated in lysine 4 (H3K4me3).

One of the by-products generated during the oxidation reaction is H₂0₂ and the detection of this was used as readout of the catalytic activity of LOXL2.

Hydrogen peroxide detection was evaluated using Amplex^{®} Red Hydrogen Peroxide/Peroxidase Assay Kit (A22188, Thermo Fisher Scientific). This assay is suitable to detect the presence of hydrogen peroxide produced from the activity of any amine oxidase.

In this detection method, H3K4me3 peptide (40 pmols) was incubated in presence of recombinant LOXL2 protein (250 ng) and different final concentrations of zonisamide (5 µM and 50 µM) in PBS buffer (137 mM NaCl, 2.7 mM KCI, 10 mM Na2HPO4 and 1.8 mM KH2PO4) for each reaction. The samples were incubated at 37° C for 5 h and fluorescence was measured by emission at 590 nm after excitation at 560 nm using a Bio-Tek fluorescent microplate reader.

The presence of Zonisamide (5 µM and 50 µM) in the reaction inhibited LOXL2 activity as seen in Figure 1. To the production of hydrogen peroxide in the absence of zonisamide was assigned arbitrarily the value 1.

### Example 2: In vivo test with breast cancer cell lines

In this in vivo test, the levels of LOXL2 and H3K4ox were checked by western-blot in a panel of breast cancer cell lines. Total extracts were obtained with 2% SDS lysis buffer (2% SDS, 50 mM Tris-HCl pH: 7.5 and 10% glycerol). Samples were keep at room temperature to avoid precipitation of the SDS, syringed five tomes, centrifuged at 13,000 rpm for 10 min and boiled at 95° C for 3 min. Protein was analysed by SDS polyacrylamide gel (SDS-PAGE) by loading the samples previously mixed with 5X loading buffer and boiled at 95° C for 3 min, transferred to nitrocellulose and incubated with the specific antibodies. For this purpose, the following cell lines were used:
- luminal A T-47D and MCF-7 cell lines (ER+/HER2-/PR+/-),
- luminal B BT-474 cell line (ER+/HER2+/PR+/-) and
- the basal breast cancer cell line MDA-MB-231 (ER-/HER2-/PR- or Triple negative, TN)
as disclosed in Holliday et al., Choosing the right cell line for breast cancer research, Breast cancer research, 2011, 13, 215.

It was observed that TN cells showed high levels of LOXL2, which in turn correlated with an enrichment of H3K4ox, the product produced by LOXL2 as shown in Figure 2.

### Example 3: In vivo test with triple negative breast cancer cell line

In this in vivo test, MDA-MB-231 cells were treated with increasing concentrations of zonisamide for several days using both a MTT assay and a clonogenic assay.

### a) MTT assay

In that MTT assay MDA-MB-231 cells (5x10⁴ cells/well) were grown in 96-well plates and exposed to different concentrations of zonisamide (0.04 mM, 0.4 mM and 4 mM). Medium was replaced every 24 h. The percentage of cell growth was determined at different time points using the 3-(4,5-dimethylthiazol-2-yl)-2,5-diphenyltetrazolium bromide (MTT) assay according to the manufacturer's instructions (M2128, Sigma). MTT is a yellowish solution and is converted to water-insoluble MTT formazan of dark blue color by mitochondrial dehydrogenases of living cells. The blue crystals are solubilized with acidified isopropanol and the intensity is measured colorimetrically at a wavelength of 570 nm.

It was observed that an increase in the concentration of zonisamide produced a reduction in the LOXL2 activity measured by the absorbance at 570 nm, as shown in Figure 3.

### b) Clonogenic assay

In that clonogenic assay, MDA-MB-231 cells were plated in a 6-well plate at a clonogenic density (10,000 cells/well) for one week. Cells were treated with zonisamide at different final concentrations (0.2 mM, 2 mM and 4 mM). Media was replaced every 24 h. For the assay, media was removed and cells were washed with PBS (137 mM NaCl, 2.7 mM KCI, 10 mM Na₂HPO₄ and 1.8 mM KH₂PO₄). Then, cells were fixed and stained with a mixture of 6% glutaraldehyde and 0.5% crystal violet for at least 30 min, as described (Franken et al., Clonogenic assay of cells in vitro, Nature Protocols, 2006, 1(5), 2315-2319). Plates were then washed with tap water, not by putting the plates under the running tap but by immersing the dishes in water carefully. Finally, plates with colonies were left to dry in normal air at room temperature.

It was observed that inhibition of LOXL2 activity decreases the proliferative capacity of these cells and causes cell death as shown in Figure 4.

## Claims

1. Zonisamide, a pharmaceutically acceptable salt thereof, a solvate thereof or a prodrug thereof, for use in an effective amount in the treatment of breast cancer.

2. Zonisamide for use according to claim 1 in the treatment of triple negative breast cancer.

3. Zonisamide for use according to claims 1 or 2, wherein zonisamide is for use alone.

4. Zonisamide for use according to claims 1 or 2, wherein zonisamide is for use in combination with other active agents.

5. Zonisamide for use according to claim 4, wherein zonisamide is for use in combination with an active agent selected from the group consisting of anastrozole, bevacizumab, capecitabine, carboplatin, cyclophosphamide, daunorubicin, denosumab, docetaxel, doxorubicin, epirubicin, epoetin alfa, eribulin, everolimus, exemestane, filgrastim, fluorouracil, fluoxymesterone, folinic acid, fulvestrant, gemcitabine, goserelin, ixabepilone, lapatinib, letrozole, leuprolide, megestrol, methotrexate, mitoxantrone, mutamycin, paclitaxel, palbociclib, pamidronate, pegfilgrastim, pertuzumab, raloxifene, tamoxifen, thiotepa, toremifene, transtuzumab, trastuzumab emtansine, triptorelin, vinblastine, vincristine, vinorelbine, zoledronic acid, pharmaceutically acceptable salts thereof, and mixtures thereof.

6. Zonisamide for use according to claim 5, wherein zonisamide is for use in combination with an active agent selected from the group consisting of bevacizumab, carboplatin, cisplatin, cyclophosphamide, docetaxel, doxorubicin, iniparib, olaparib, paclitaxel, pharmaceutically acceptable salts thereof, and mixtures thereof.

7. Pharmaceutical composition comprising an effective amount of zonisamide, a pharmaceutically acceptable salt thereof, a solvate thereof or a prodrug thereof, and at least one pharmaceutically acceptable excipient or vehicle, for use in the treatment of breast cancer.

8. Pharmaceutical composition for use according to claim 7, for use in the treatment of triple negative breast cancer.

9. Pharmaceutical composition for use according to claims 7 or 8, wherein zonisamide is for use in combination with an additional active agent selected from the group consisting of anastrozole, bevacizumab, capecitabine, carboplatin, cisplatin, cyclophosphamide, daunorubicin, denosumab, docetaxel, doxorubicin, epirubicin, epoetin alfa, eribulin, everolimus, exemestane, filgrastim, fluorouracil, fluoxymesterone, folinic acid, fulvestrant, gemcitabine, goserelin, iniparib, ixabepilone, lapatinib, letrozole, leuprolide, megestrol, methotrexate, mitoxantrone, mutamycin, olaparib, paclitaxel, palbociclib, pamidronate, pegfilgrastim, pertuzumab, raloxifene, sorafenib, sunitinib, tamoxifen, thiotepa, toremifene, transtuzumab, trastuzumab emtansine, triptorelin, vinblastine, vincristine, vinorelbine, zoledronic acid, pharmaceutically acceptable salts thereof, and mixtures thereof.

10. Pharmaceutical composition for use according to claim 9, wherein zonisamide is for use in combination with an additional active agent selected from the group consisting of bevacizumab, carboplatin, cisplatin, cyclophosphamide, docetaxel, doxorubicin, iniparib, olaparib, paclitaxel, pharmaceutically acceptable salts thereof, and mixtures thereof.

11. Pharmaceutical composition for use according to claims 9 or 10, wherein zonisamide and the additional active agent are for simultaneous, separate or sequential use.


SEQUENCE LISTING

<110>  Fundació Institut Mar d\222Investigacions Mèdiques

<120>  Zonisamide for use in the treatment of breast cancer

<130>  IMIM_01

<160>  1

<170>  PatentIn version 3.5

<210>  1
<211>  16
<212>  PRT
<213>  SYNTHETIC SEQUENCE

<400>  1

Ala Arg Thr Lys Gln Thr Ala Arg Lys Ser Thr Gly Gly Lys Ala Pro
1               5                   10                  15